**Europäisches Patentamt**

**European Patent Office**

**Office européen des brevets**

(19)

(11) Veröffentlichungsnummer: **0 377 430**
**A1**

(12) # EUROPÄISCHE PATENTANMELDUNG

(21) Anmeldenummer: **89890323.2**

(22) Anmeldetag: **18.12.89**

(51) Int. Cl.⁵: **C07C 51/47, C07C 59/08,**
**C07C 59/255, C07C 59/265**

(30) Priorität: **05.01.89 AT 23/89**
**06.12.89 AT 2773/89**

(43) Veröffentlichungstag der Anmeldung:
**11.07.90 Patentblatt 90/28**

(84) Benannte Vertragsstaaten:
**BE DE ES FR GB IT NL**

(71) Anmelder: **JUNGBUNZLAUER**
**AKTIENGESELLSCHAFT**
**Schwarzenbergplatz 16**
**A-1010 Wien(AT)**

(72) Erfinder: **Edlauer, Robert**
**Försterweg 23**
**A-2136 Laa/Thaya(AT)**
Erfinder: **Kirkovits, August E., Dipl.-Ing.**
**Stronegg 21**
**A-2153 Stronsdorf(AT)**
Erfinder: **Westermayer, Ronald, Dr.**
**Försterweg 29**
**A-2136 Laa/Thaya(AT)**
Erfinder: **Stojan, Otto, Dipl.-Ing.**
**Försterweg 25**
**A-2136 Laa/Thaya(AT)**

(74) Vertreter: **Collin, Hans, Dipl.-Ing. Dr. et al**
**Patentanwälte Dipl.-Ing. Dr. Hans Collin**
**Dipl.-Ing. Erwin Buresch Dipl.-Ing.Armin**
**Häupl Mariahilferstrasse 50**
**A-1070 Wien(AT)**

(54) **Verfahren zur Abtrennung von Säuren aus salz- und kohlenhydrathaltigen Substraten.**

(57) Vorgeschlagen wird ein Verfahren zur Abtrennung von Säuren aus salz-und kohlehydrathaltigen Substraten, bei denen die Substrate über Harze geleitet werden, die die Säuren adsorptiv aus dem Substrat entfernen, und anschließend die Säuren durch Elution von den Harzen abgetrennt werden, mit der Besonderheit, daß als Harze basische Ionentauscherharze eingesetzt werden.

EP 0 377 430 A1

Die Erfindung betrifft ein Verfahren zur Abtrennung von Säuren aus salz-und kohlehydrathaltigen Substraten, bei denen die Substrate über Harze geleitet werden, die die Säuren adsorptiv aus dem Substrat entfernen, und anschließend die Säuren durch Elution von den Harzen abgetrennt werden.

In vielen Industriezweigen werden organische und anorganische Säuren verwendet: bei Applikationen im Pharma- und Lebensmittelbereich, aber auch beim Einsatz in Reinigungschemikalien, müssen diese eine besonders hohe Qualität aufweisen.

Als Reinigungsmethoden für Säuren sind neben der Fällung als Schwermetall bzw. Erdalkalisalz auch die Flüssig/Flüssig-Extraktion und die Bildung von flüchtigen Derivaten (z.B. Methylester) vorgeschlagen worden. Alle diese Methoden finden bei der Aufbereitung von technisch wichtigen Säuren wie z.B. Milchsäure, Äpfelsäure, Bernsteinsäure, Weinsäure, Zitronensäure, Gluconsäure und Phosphorsäure auch großtechnische Anwendung. Die erwähnten Verfahren haben jedoch schwerwiegende Nachteile. Die Reinigungsmethoden, welche die Säure über ein unlösliches Salz isolieren, verbrauchen große Mengen an Hilfschemikalien. In den meisten Fällen wird ein schwerlösliches Calziumsalz gebildet, welches anschließend mit Schwefelsäure zur freien Säure und einer äquimolaren Menge Gips umgesetzt wird. Neben den Kosten für Kalk und Schwefelsäure muß daher auch noch der dabei anfallende Gips entsorgt werden. Reinigungsmethoden basierend auf einer Verteilung zwischen zwei ineinander unlöslichen Phasen, und auch Verfahren, welche die Säure zu einem leichter flüchtigen Derivat umsetzen, arbeiten mit großen Mengen organischer Lösungsmittel, die ein Sicherheits- und Umweltrisiko darstellen.

Die Extraktion von Milchsäure ist in der EP 0 159 585 beschrieben; die DE-OS 32 22 837 stellt ein Verfahren, welches Milchsäure durch Bildung von Methyllactate reinigt, vor.

Methoden zur Reinigung von Zitronensäure mittels Flüssig/Flüssig-Extraktion sind bereits in den US-PSen 4 251 671 und 4 334 095 sowie in der AT-PS 382 364 vorgeschlagen.

Ein Verfahren zur Isolierung von Zitonensäure aus Fermentationsbrühen durch Bildung schwerlöslicher Salze ist in der US-PS 2 810 755 beschrieben.

Weitere Verfahren zur Extraktion von Weinsäure bzw. Zitronen-, Äpfel-und Milchsäure wurden in der DE-OS 1 443 538 und der EP 0 049 429 vorgeschlagen.

Reinigungsmethoden für die Phosphorsäure sind in den DE-OSen 23 20 877 und 17 67 442 sowie in der DE-AS 23 21 751 beschrieben.

Für Zitronensäure ist in der EP-A 0 151 470 noch ein Verfahren beschrieben, bei dem aus der unreinen Zitronensäurelösung zuerst höhermolekulare Verbindungen durch Ultrafiltration abgetrennt werden; anschließend werden noch vorhandene Verunreinigungen durch Adsorption an nichtionogenen Harzen großer spezifischer Oberfläche entfernt, wobei die wesentlich polarere Zitronensäure nicht gebunden wird.

Als weitere Reinigungsmöglichkeit beschreibt die US-PS 4 720 579 die Adsorption der Zitronensäure an neutralen, nichtionogenen, makroretikularen wasserunlöslichen Harzen. Als Eluens wird Wasser oder eine Wasser-Acetonmischung eingesetzt. Das Verfahren ist in der Lage, Salze und Kohlenhydrate von der Zitronensäure abzutrennen.

Die vorliegende Erfindung geht vom Stand der Technik aus, der durch die US-PS 4 720 579 gegeben ist. Dabei geht es grundsätzlich darum, Substrate, die einen Gehalt an Säuren, Salzen und Kohlehydraten aufweisen, über Harze zu leiten, die selektiv als Adsorber für die Säuren wirken, während die anderen Bestandteile der Substrate durchgehen. Die Säuren, um deren Reinigung bzw. Gewinnung es geht, werden anschließend durch Elution der beladenen Harze gewonnen.

Es wurde nunmehr überraschenderweise gefunden, daß basische Ionentauscherharze geeignet sind zur chromatographischen Isolierung und/oder Reinigung von Säuren generell, d.h. sowohl von organischen als auch von anorganischen Säuren, wobei von den Carbonsäuren insbesondere die Fruchtsäuren zu erwähnen sind. Von den typischen sauren organischen Genußsäuren werden die Weinsäure, Zitronensäure, Äpfelsäure, Fumarsäure und Bernsteinsäure in der Literatur üblicherweise als Fruchtsäuren bezeichnet.

Die Säuren werden selektiv und reversibel adsorbiert und die erzielten Reinigungswirkungen und die Beladbarkeit der Harze sind wesentlich höher als die der nichtionogenen Harze.

Demgemäß ist das erfindungsgemäße Verfahren zur chromatographischen Reinigung oder Trennung von Säuren vor allem dadurch gekennzeichnet, daß zur Trennung basische Ionenaustauscherharze verwendet werden.

Die Verwendung von basischen Ionentauscherharzen bei der Reinigung von Fruchtsäuren ist an sich bekannt bei Verfahren, bei denen die Fruchtsäuren selbst durch das Ionentauscherbett hindurchgehen, während Verunreinigungen im Substrat, wie stärkere Säuren oder Salze, durch Ionentausch am Harz festgehalten werden. Die Ionentauscherharze werden bei diesen bekannten Verfahren vor dem Durchbruch der Verunreinigungen regeneriert, wobei die Beladung entfernt wird.

So beschreibt die DD-PS 203 533 ein Ionentauschverfahren zur Gewinnung von Carbon- und Oxycarbonsäuren aus deren fremdsalzhaltigen Salzlösungen, bei dem die Salze zuerst über Kationentauscher in der H$^+$-Form in Säuren umgewandelt werden. Dabei wird ein Gemisch aus Carbonsäuren und Fremdsäuren erhalten. Mit einer geringer konzentrierten Fraktion dieses Gemisches wird sodann ein vorerst in der Basen- oder Hydroxylform vorliegender Anionentauscher beladen, also im wesentlichen in die Carbonsäureform übergeführt. Anschließend wird über den säurebeladenen Ionentauscher eine höher konzentrierte Fraktion des in der Entkationisierungsstufe erhaltenen Säuregemisches geleitet, wobei die zu gewinnende Säure das Harzbett ungehindert passiert und lediglich die stärkeren Fremdanionen (erwähnt ist Chlorid) durch Ionentausch an das Harz gebunden werden.

Die DE-OS 29 31 759 betrifft die Abtrennung von Zitronensäure aus einem Gemisch mit Oxalsäure und Salpetersäure, bei dem dieses Gemisch über einen Anionentauscher geleitet wird, der gegebenenfalls vorher mit Zitronensäure äquilibriert worden ist. Der Ionentauscher holt aus diesem Gemisch die Salpetersäure und die Oxalsäure durch Ionentausch heraus, während die Zitronensäure die Säule passiert.

Die DE-OS 25 43 332 betrifft ein Verfahren zur Abtrennung der Maleinsäureverunreinigung aus synthetischer Weinsäure, bei dem diese über basische Anionentauscher, die in Hydroxyl- oder Tartratform vorliegen, geleitet wird. Wiederum holt der Ionentauscher durch Ionentausch die stärkere Maleinsäure aus dem Gemisch heraus, die zu geweinnende Weinsäure passiert die Säule.

Beim erfindungsgemäßen Verfahren werden die zu gewinnenden bzw. reinigenden Säuren nicht durch Ionentausch, sondern adsorptiv an die Harze gebunden und von dort durch Elution gewonnen.

Vorteilhaft werden die basischen Ionentauscherharze zur Isolierung und/oder Reinigung von Säuren mit einem pK$_S$-Wert im Bereich von 1,2 bis 4,8, vorzugsweise von 2,1 vis 3,9, eingesetzt.

Vorzugsweise werden die Harze in säurebeladener Form eingesetzt. Die Beladung des Harzes erfolgt erfindungsgemäß vorteilhaft mit einem Anion, zu dem das Harz die gleiche oder eine höhere Affinität als zu der zu reinigenden Säure aufweist. Die Beladung des Harzes muß dabei nicht als explizite Operation durchgeführt werden, in vielen Fällen stellt sie sich durch längeren Gebrauch von selbst ein. Insbesondere werden mit Salz-, Salpeter-, Phorphor-, Zitronen- oder Schwefelsäure, vorzugsweise mit Schwefelsäure, beladene basische Ionentauscherharze eingesetzt.

Die erfindungsgemäßen Anionentauscherharze können stark oder auch schwach basischer Natur sein gelförmig oder makroretikular aufgebaut sein. U.a. können die Ionentauscher eine Styrol/Divinylbenzolbasis oder eine vernetzte Acrylharzbasis aufweisen.

Die Elution kann, wie bekannt, mit Wasser erfolgen; vorzugsweise wird jedoch eine verdünnte Säure, insbesondere Schwefelsäure in einem Konzentrationsbereich von 0 bis 1 %, eingesetzt. Wie bekannt, können auch beim erfindungsgemäßen Verfahren günstig niedrige pH-Werte eingestellt werden. Die Elutionstemperatur kann zwischen Raumtemperatur und der Stabilitätsgrenztemperatur des Harzes gewählt werden, vorzugsweise erfolgt die Elution bei einer Temperatur, welche mikrobielle Kontaminationen des Harzes verhindert.

Das Verfahren wird vorzugsweise in einer quasikontinuierlichen Apparatur (simulated moving bed) durchgeführt, vollkontinuierliche oder auch batch-Fahrweisen sind jedoch auch möglich.

Die Erfindung wird im folgenden an Hand von Beispielen erläutert. Die dabei verwendeten Harze sind übliche Handelsprodukte laut nachstehender Tabelle:

| Name | Polymer-Basis | akt.Gruppe | Typ | Hersteller |
|---|---|---|---|---|
| AMBERLITE XAD-4 | Polystyrol | - | 0 | Rohm&Haas |
| AMBERLITE IRA904 | Styrol/DVB | Amin | --- | Rohm&Haas |
| AMBERLITE IRA900 | Styrol/DVB | Amin | --- | Rohm&Haas |
| AMBERLITE IRA958 | Acryl/DVB | Amin | --- | Rohm&Haas |
| AMBERLITE IRA93 | Styrol/DVB | Amin | - | Rohm&Haas |
| AMBERLITE IRA67 | Acryl | Amin | - | Rohm&Haas |
| AMBERLITE IRA35 | Acryl/DVB | Amin | - | Rohm&Haas |
| LEWATIT MP64 | Polystyrol | Amin | - | Bayer |
| LEWATIT MP500A | Polystyrol | Amin | -- | Bayer |
| DOWEX WGR2 | Epoxyamin | Amin | - | Dow Chemical |
| RELITE MG1 | Acryl/DVB | Polyamin | - | Tanatex |
| 0 .... nicht ionogen | | | | |
| - .... schwach basisch | | | | |
| -- .... begrenzt stark basisch | | | | |
| --- .... stark basisch | | | | |

Beispiel 1:

Die Anionentauscherharze wurden nach einer vollständigen Regeneration mittels 4 %iger NaOH mit 10 %iger Schwefelsäure in die Sulfatform gebracht und mit 0,1 %iger Schwefelsäure konditioniert. Die so vorbereiteten Harze wurden in Säulen von 25 mm Durchmesser und einer Länge von 800 mm gefüllt. Nach der Injektion von 5 ml einer Lösung, bestehend aus 40 % Zitronensäure und 10 % Maltose in Wasser, wurde mit 15 ml/min 0,1 %iger Schwefelsäure eluiert. Die Säulentemperatur wurde mit einem Umwälzthermostat auf 80°C eingestellt. Die Eluatkonzentrationen wurden durch Messung des Brechungsindex bestimmt. Unter diesen Bedingungen wurde Maltose und Zitronensäure auf fast allen Harzen vollkommen getrennt. Die Tabelle zeigt die Retentionsvolumina von Maltose und Zitonensäure.

| Harz | Elutionsvolumen Maltose | Elutionsvolumen Zitronensäure |
|---|---|---|
| | [ml] | [ml] |
| AMBERLITE IRA 904 | 285 | 505 |
| AMBERLITE IRA 900 | 270 | 911 |
| AMBERLITE IRA 958 | 270 | 656 |
| AMBERLITE IRA 93 SP | 338 | 806 |
| AMBERLITE IRA 67 | 210 | 566 |
| AMBERLITE IRA 35 | 296 | 544 |
| LEWATIT MP 64 | 341 | 731 |
| LEWATIT MP 500A | 251 | 690 |
| DOWEX WGR 2 | keine Trennung möglich | |
| RELITE MG 1 | keine Trennung möglich | |

Beispiel 2:

Wie im Beispiel 1 wurde eine Säule mit AMBERLITE IRA 67 gepackt und mit einem Fluß von 30 ml/min mit 0,1 %iger Schwefelsäure äquilibriert. Die Temperatur der Säule wurde auf 75°C eingestellt. Das Ausschlußvolumen des Harzes wurde durch Injektion von 1 ml einer 5 %igen Dextran T10-Lösung bestimmt. Es kann angenommen werden, daß ein hochmolekularer, ungeladener Stoff unter diesen Bedingungen nicht retendiert wird. Das Ausschlußvolumen wurde wie folgt berechnet:

$$e = \frac{T_{tr}}{\dfrac{V}{Fl}}$$

$T_{tr}$    Retentionszeit Dextran in Sekunden

V    Säulenvolumen in ml

Fl    Fluß in $ml.s^{-1}$

Mittels eines Schleifinjektors wurde nun genau 1 ml verschiedener Substanzen in den Laufmittelstrom eingebracht und am Säulenaustritt über den Brechungs index detektiert. Gemessen wurde die Zeit bis zum Auftreten der maximalen Konzentration im Eluat und die Breite des Peaks in halber Höhe. Aus diesen Daten wurde die Trennstufenzahl und der Verteilungskoeffizient berechnet:

$$T_{max} = T - T_{er}$$

$$n = 16 * \ln(2) * \frac{T_{max}^2}{d^2}$$

$$K = \frac{u * T_{max}}{(1-e) * z}$$

T    Retentionszeit der Substanz in Sekunden
n    Trennstufenzahl der Säule
d    Breite des Substanzpeaks bei halber Höhe in Sekunden
u    Flächenbezogener Fluß in ml/s.cm$^2$
z    Säulenlänge in cm
K    Verteilungskoeffizient der Substanz

Die folgende Tabelle zeigt die dabei erhaltenen Resultate:

| Substanz | Konzentration | Trennstufenzahl | Verteilungskoeffizient |
|---|---|---|---|
| | [Gewichts %] | n | K |
| Natriumsulfat | 10 | 9.09 | 0.219 |
| | 30 | 10.11 | 0.262 |
| Maltose | 10 | 2.25 | 0.149 |
| | 20 | 2.17 | 0.146 |
| | 30 | 2.25 | 0.149 |
| | 40 | 2.25 | 0.149 |
| Glucose | 10 | 6.24 | 0.362 |
| | 20 | 6.02 | 0.356 |
| | 40 | 5.81 | 0.349 |
| Gluconsäure | 10 | 6.46 | 0.541 |
| | 20 | 6.22 | 0.514 |
| | 30 | 6.22 | 0.514 |
| Milchsäure | 10 | 15.11 | 0.673 |
| | 40 | 14.07 | 0.663 |
| Zitronensäure | 10 | 17.50 | 1.864 |
| | 20 | 18.06 | 1.815 |
| | 30 | 16.48 | 1.748 |
| | 40 | 17.25 | 1.775 |

Aus diesen Werten erkennt man einwandfrei, daß eine Reinigung von Zitronensäure unter den gewählten Bedingungen möglich ist. Die Daten zeigen auch eine Trennung von Zitronensäure - Gluconsäure und zwischen Zitronensäure - Milchsäure.

Beispiel 3:

Beispiel 2 wurde bei einer Temperatur von 60°C wiederholt.

**EP 0 377 430 A1**

| Substanz | Konzentration | Trennstufenzahl | Verteilungskoeffizient |
|---|---|---|---|
| | [Gewichts % ] | n | K |
| Natriumsulfat | 10 | 7.70 | 0.192 |
| | 30 | 8.71 | 0.242 |
| Maltose | 10 | 1.62 | 0.110 |
| | 40 | 1.70 | 0.112 |
| Glucose | 10 | 4.93 | 0.322 |
| | 40 | 4.57 | 0.312 |
| Gluconsäure | 10 | 5.33 | 0.489 |
| | 40 | 4.85 | 0.483 |
| Zitronensäure | 10 | 13.83 | 1.743 |
| | 40 | 12.69 | 1.617 |

Beispiel 4:

Beispiel 2 wurde mit einer Temperatur von 90 °C wiederholt.

| Substanz | Konzentration | Trennstufenzahl | Verteilungskoeffizient |
|---|---|---|---|
| | [Gewichts % ] | n | K |
| Natriumsulfat | 10 | 11.09 | 0.227 |
| | 30 | 12.39 | 0.280 |
| Maltose | 10 | 2.97 | 0.183 |
| | 40 | 3.06 | 0.186 |
| Glucose | 10 | 8.76 | 0.404 |
| | 40 | 8.22 | 0.392 |
| Gluconsäure | 10 | 10.96 | 0.628 |
| | 40 | 9.80 | 0.594 |
| Milchsäure | 10 | 18.99 | 0.776 |
| | 40 | 17.77 | 0.752 |
| Zitronensäure | 10 | 30.00 | 2.514 |
| | 40 | 25.10 | 2.147 |

Vergleicht man die Resultate aus Beispiel 3 und Beispiel 4, so erkennt man den günstigen Einfluß der Temperatur auf die Trennung.

Beispiel 5:

Eine Polypropylensäule mit einem Durchmesser von 27 mm und einer Länge von 146 mm wurde mit Amberlite IRA-67 in der Sulfatform gepackt. Bei einer Temperatur von 28 °C wurden 4,97 ml/min 0,1 %ige Schwefelsäure über die Säule gepumpt. In den Laufmittelstrom wurden dann 0,5 ml Flußsäure bzw. Phosphorsäure injiziert. Das Austreten der Substanzen aus der Säule wurde mittels eines Leitfähigkeitsdetektors gemessen.

6

| Substanz | Konzentration | Trennstufenzahl | Verteilungskoeffizient |
|---|---|---|---|
| | [Gewichts % ] | n | K |
| Flußsäure | 10 | 15.45 | 1.06 |
| Phosphorsäure | 40 | 15.01 | 3.14 |

Dieses Beispiel demonstriert die Möglichkeit, auch anorganische Säuren zu reinigen.

Beispiel 6:

Beispiel 2 wurde mit 0,1 %iger Schwefelsäure als Laufmittel und IRA900 als Ionentauscher bei einer Temperatur von 75° C wiederholt.

| Substanz | Konzentration | Trennstufenzahl | Verteilungskoeffizient |
|---|---|---|---|
| | [Gewichts % ] | n | K |
| Natriumsulfat | 10 | 6.80 | 0.319 |
| | 30 | 8.01 | 0.358 |
| Maltose | 10 | 2.77 | 0.316 |
| | 40 | 2.77 | 0.309 |
| Glucose | 10 | 6.24 | 0.485 |
| | 40 | 6.24 | 0.485 |
| Gluconsäure | 10 | 7.02 | 0.726 |
| | 40 | 6.43 | 0.717 |
| Milchsäure | 10 | 16.73 | 1.036 |
| | 40 | 16.41 | 1.043 |
| Weinsäure | 10 | 19.74 | 2.909 |
| Äpfelsäure | 10 | 16.86 | 2.212 |
| Itaconsäure | 10 | 15.96 | 3.095 |

Beispiel 7:

Beispiel 2 wurde mit Wasser als Laufmittel und IRA900 als Ionentauscher bei einer Temperatur von 75° C wiederholt.

| Substanz | Konzentration | Trennstufenzahl | Verteilungskoeffizient |
|---|---|---|---|
| | [Gewichts % ] | n | K |
| Natriumsulfat | 10 | 7.70 | 0.343 |
| | 30 | 7.90 | 0.370 |
| Maltose | 10 | 3.09 | 0.340 |
| | 40 | 2.96 | 0.340 |
| Glucose | 10 | 7.62 | 0.545 |
| | 40 | 7.03 | 0.534 |
| Milchsäure | 40 | 35.47 | 2.549 |

Beispiel 8:

Eine halbkontinuierliche Chromatographieanlage bestehend aus 10 Säulen (Durchmesser 25 mm, Länge 1600 mm) wurde mit AMBERLITE IRA 900 in der Suflatform gefüllt. Die Anlage ist so aufgebaut, daß die Säulen durch Rohrleitungen zu einem Ring verbunden sind. Diese Ringleitung ist zwischen den Säulen über Ventile an vier Versorgungsleitungen angeschlossen. Diese Versorgungsleitungen sind Rohprodukteintritt, Eluenseintritt, Produktauslaß und Raffinatauslaß. Die Flüsse in der Anlage werden mittels regelbarer Kolbenpumpen konstant gehalten. In diesem Prozeß sind immer genau vier Ventile geöffnet und teilen den aus Säulen gebildeten Ring in vier Zonen. Diese Zonen werden durch einen Prozeßrechner nach vorgegebenen Zeiten um eine Säule in Flußrichtung weiter geschalten. Diese Schaltvorgänge simulieren einen Harzfluß gegen die Flußrichtung, in der Literatur wird er deswegen als "simulated moving bed"-Prozeß bezeichnet.

Die Zoenaufteilung erfolgte nach folgendem Schema:

| Zone 1 | 2 Säulen |
|--------|----------|
| Zone 2 | 3 Säulen |
| Zone 3 | 4 Säulen |
| Zone 4 | 1 Säule  |

Pro Minute wurden 8 ml Rohprodukt und 32 ml Wasser eingespeist und 16 ml Produkt bzw. 24 ml Raffinat entnommen. Die Temperatur der Anlage wurde auf 70 °C konstant gehalten. Die interne Umwälzmenge im Säulenring wurde auf 4 ml/min eingestellt. Die Umschaltung der Ventile erfolgte alle 1550 Sekunden. In diese Anlage wurde eine auf etwa 40 % aufkonzentrierte Milchsäurefermentationsmaisce eingespeist. Dabei wurde eine gereinigte Milchsäure mit einer Ausbeute von über 95 % gewonnen. Das Raffinat enthielt neben Milchsäurespuren, Farbstoffen, Kohlehydraten und Salzen vor allem Polymere, welche die Milchsäureproduktion empfindlich stören können. Der Reinigungseffekt wird auch im hohen chemischen Sauerstoffbedarf des Raffinats von 13,5 g/l dargestellt. In der folgenden Tabelle wurde die Milchsäure vor und nach der chromatographiscehn Trennung verglichen:

| | vor | nach |
|---|---|---|
| Milchsäure [%] | 40.52 | 19.53 |
| Farbe [405nm.1cm] | 1.94 | 0.43 |
| Sulfat [%] | 1.78 | 0.03 |
| Chlorid [mg/kg] | 330 | 23 |
| Natrium [mg/kg] | 7820 | 330 |
| Magnesium [mg/kg] | 7 | 0 |
| Eisen [mg/kg] | 27 | 5 |

Beispiel 9:

Eine halbtechnische Chromatographieanlage, bestehend aus zwölf Säulen (Durchmesser 0,5 m, Länge 2,0 m) wurde mit Amberlite IRA-67 gefüllt. Das Harz wurde durch Spülen mit 0,1 %iger Schwefelsäure in die Sulfatform gebracht. Die Säulen wurden wie im nachstehenden Schema ersichtlich verbunden.

Bei einer Temperatur von 75 °C wurden 215 l/h einer auf ca. 45 Gew.-% ankonzentrierten Zitronensäurefermentationsbrühe und 825 l/h 0,1 %ige Schwefelsäure als Elutionsmittel zugeführt. Gleichzeitig wurden 500 l/h gereinigte Zitronensäurelösung (Produkt) und 533 l/h einer Lösung, welche die Verunreinigungen enthält (Raffinat) entnommen. Im Säulenring wurden 192 l/h umgewälzt, die Ein- bzw. Auslaßstellen wurden alle 1800 s um eine Säule in Flußrichtung weitergeschaltet.

Die Analyse der zu- und abgeführten Lösungen ergab folgende Werte:

|  | Fermentationsbrühe | Produkt | Raffinat |
|---|---|---|---|
| Zitronensäure | 43.45% | 22.36% | 0.02% |
| Glucose | 0.25% | 0.00% | 0.16% |
| Fructose | 0.10% | 0.00% | 0.11% |
| Maltose + Isomaltose | 1.40% | 0.00% | 0.80% |
| Gesamtzucker | 4.60% | 0.03% | 2.35% |
| Sulfat | 0.98% | 0.27% | 0.43% |
| Chlorid | 110ppm | 16ppm | 42ppm |
| Eisen | 8ppm | 2ppm | 2ppm |
| Kalium | 370ppm | 0ppm | 190ppm |
| Natrium | 55ppm | 0ppm | 29ppm |
| Calzium | 20ppm | 1ppm | 8ppm |
| Magnesium | 120ppm | 0ppm | 60ppm |
| Dichte | 1.27g/ml | 1.07g/ml | 1.00g/ml |

Die Produktivität der Anlage beträgt 2,86 t Zitonensäureanhydrat pro Tag, die Ausbeute ist mit 99,9 % wesentlich besser als die der bereits bekannten Reinigungsverfahren für Zitronensäure. Man erkennt deutlich, daß nahezu alle Verunreinigungen, egal ob kationisch, anionisch oder ungeladen, abgetrennt werden.

Beispiel 10:

Beipsiel 9 wurde mit Rohphosphorsäure wiederholt. Eingespeist wurde eine vorfiltrierte Naßphosphorsäure (Grünsäure) mit einer Konzentration vib 37 % $P_2O_5$. Die Säulenumschaltung erfolgte im Abstand von 1500 Sekunden. Die Analyse der Produkt- und Raffinatproben im Gleichgewichtszustand zeigte folgende Werte:

|  | Produkt | Raffinat |
|---|---|---|
| titrierbare Säure als $P_2O_5$ | 15.60 | 1.90 % |
| Farbe (405nm. 1cm) | 0.047. | 1.486 |

Die Phosphorsäureausbeute betrug 89,9 %, ein Vergleich der Kationenbelastung vor und nach der chromatographischen Trennung zeigt die hohe Reinigungsleistung des Systems (alle Konzentrationen bezogen auf 100 % $P_2O_5$).

|  | vor | nach |
|---|---|---|
| Magnesium | 20800 | 42 mg/kg |
| Calzium | 358 | 9 mg/kg |
| Aluminium | 3390 | 410 mg/kg |
| Eisen | 4620 | 1200 mg/kg |
| Zink | 855 | 15 mg/kg |
| Chrom | 614 | 102 mg/kg |
| Kupfer | 29 | 0 mg/kg |
| Nickel | 56 | 0 mg/kg |
| Mangan | 21 | 0 mg/kg |
| Cadmium | 23 | 0 mg/kg |

**Ansprüche**

1. Verfahren zur Abtrennung von Säuren aus salz- und kohlehydrathaltigen Substraten, bei denen die Substrate über Harze geleitet werden, die die Säuren adsorptiv aus dem Substrat entfernen, und anschließend die Säuren durch Elution von den Harzen abgetrennt werden, dadurch gekennzeichnet, daß als Harze basische Ionentauscherharze eingesetzt werden.

2. Verfahren nach Anspruch 1, dadurch gekennzeichnet, daß die basischen Ionentauscherharze zur Isolierung und/oder Reinigung von anorganischen Säuren eingesetzt werden.

3. Verfahren nach Anspruch 1, dadurch gekennzeichnet, daß die basischen Ionentauscherharze zur Isolierung und/oder Reinigung von organischen Säuren eingesetzt werden.

4. Verfahren nach Anspruch 3, dadurch gekennzeichnet, daß die basischen Ionentauscherharze zur Isolierung und/oder Reinigung von Fruchtsäuren eingesetzt werden.

5. Verfahren nach einem der Ansprüche 2 bis 4, dadurch gekennzeichnet, daß die basischen Ionentauscherharze zur Isolierung und/oder Reinigung von Säuren mit einem $pK_S$-Wert im Bereich von 1,2 bis 4,8, vorzugsweise von 2,1 bis 3,9, eingesetzt werden.

6. Verfahren nach einem der Ansprüche 1 bis 5, dadurch gekennzeichnet, daß säurebeladene basische Ionentauscherharze eingesetzt werden.

7. Verfahren nach Anspruch 6, dadurch gekennzeichnet, daß Ionentauscherharze eingesetzt werden, die mit einer Säure beladen sind, zu der sie eine höhere Affinität aufweisen als zur zu isolierenden und/oder zu reinigenden Säure.

8. Verfahren nach Anspruch 6 oder 7, dadurch gekennzeichnet, daß mit Salz-, Salpeter-, Phosphor-, Zitronen- oder Schwefelsäure, vorzugsweise mit Schwefelsäure, beladene basische Ionentauscherharze eingesetzt werden.

9. Verfahren nach einem der Ansprüche 1 bis 8, dadurch gekennzeichnet, daß als Eluens verdünnte Säure eingesetzt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-2 697 725 (BRYCE)<br>* Ansprüche 1-13; Spalte 4, Zeile 42 - Spalte 5, Zeile 1 *<br>--- | 1,3-9 | C 07 C 51/47<br>C 07 C 59/08<br>C 07 C 59/255<br>C 07 C 59/265 |
| X | US-A-2 664 441 (OWENS et al.)<br>* Ansprüche 1-6; Spalte 2, Zeilen 1-13 *<br>--- | 1,3,4,6 -9 | |
| X | CH-A- 523 861 (SUN OIL CO.)<br>* Patentanspruch; Unteransprüche 1-3; Spalte 3, Zeilen 45-56; Spalte 6, Zeilen 4-8; Spalte 8, Beispiel 1 *<br>--- | 1,3,6 | |
| A | FR-A-1 194 165 (ETABLISSEMENTS E. DEGREMONT)<br>* Ganzes Dokument *<br>--- | 1 | |
| A | CHEMICAL ABSTRACTS, Band 83, Nr. 23, 5. Juni 1978, Zusammenfassung Nr. 169603b, Columbus, Ohio, US; & JP-A-77 156 814 (TOKUYAMA SODA CO., LTD) 27-12-1977<br>----- | 1 | |
| | | | **RECHERCHIERTE SACHGEBIETE (Int. Cl.5)**<br><br>C 07 C 51/00<br>C 07 C 59/00 |

Der vorliegende Recherchenbericht wurde für alle Patentansprüche erstellt

| Recherchenort | Abschlußdatum der Recherche | Prüfer |
|---|---|---|
| DEN HAAG | 03-04-1990 | KLAG M.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................................
& : Mitglied der gleichen Patentfamilie, übereinstimmendes Dokument

EPO FORM 1503 03.82 (P0403)